Europäisches Patentamt

European Patent Office

Office européen des brevets

(11) Publication number: **0 210 321**
**A1**

## EUROPEAN PATENT APPLICATION

(21) Application number: 85830190.6

(51) Int. Cl.4: **A61N 5/00**

(22) Date of filing: 23.07.85

(43) Date of publication of application:
04.02.87 Bulletin 87/06

(84) Designated Contracting States:
AT BE CH DE FR GB LI LU NL SE

(71) Applicant: Fassina, Antonio
Via Lecco, 2
I-20100 Milano(IT)

(72) Inventor: Fassina, Antonio
Via Lecco, 2
I-20100 Milano(IT)

(54) Pain-relieving composite having a relatively rapid action.

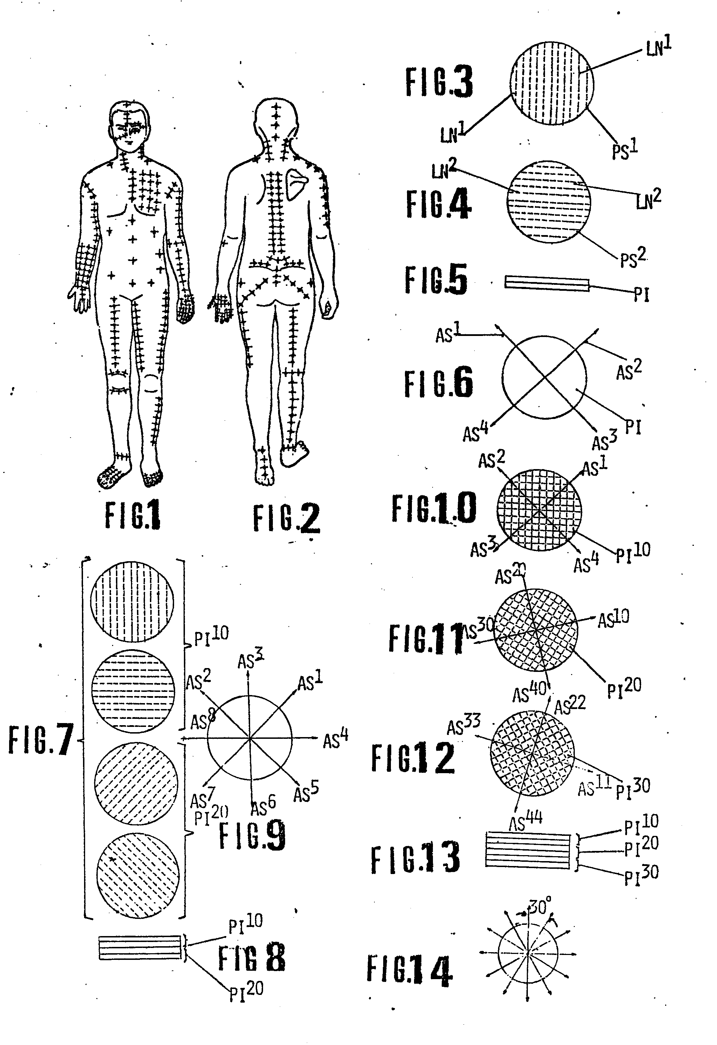

(57) A movable composite is provided consisting of pairs of polarizing plates Fig 3, 45 having their polarization axes being perpendicular to one another, the composite being for application to the human body in order to relieve pain.

The composite is considered to exploit terrestrial magnetism and/or other radiation present in the earth.

FIG.3

FIG.4

FIG.5

EP 0 210 321 A1

# PAIN-RELIEVING COMPOSITE HAVING A RELATIVELY RAPID ACTION

The present invention relates to a pain-relieving composite having a relatively rapid action.

In European Patent No. 0065473 a device is described comprising plates of Polaroid (trade mark) film, which device is useful for the treatment of the "reflexed" podalic portions of the human body in order to decrease the effects of vertebral - (rhachidian) derangements.

In essence, the disclosed device consists of two Polaroid plates oriented at right angles to each other and incorporated into an "under-foot" structure attached to a shoe sole so that one of the bisecting lines of the polarization axes of the plates coincides with the orthopaedic (i.e. optimum) axis of the foot.

According to the patent, the described device decreases the effects arising from rhachidian derangements, the action of the device acting upon the whole body and requiring from between a few days to some months to be appreciated. The inventor, Bernard Bricot, does not provide any explanation of the mode of action of his disclosed two-component device, and has limited the application of his device to the purpose set out in the patent, without suggesting any extension of the applicability of his device and without indicating how any drawbacks of his device may be obviated.

Through more detailed study of the mode of action of devices of the type disclosed in that patent (as will be more fully described hereinafter), it has been concluded that such devices can produce a strong as well as relatively rapid pain-relieving action if directly applied to the site of the pain. Also the pain-relieving action is found to be maximised if the active axes defined by the bisecting lines of the polarization axes of the plates coincide with the orthopaedic axes of the painful site.

According to the present invention there is provided a pain-relieving composite having a relatively rapid action, characterised in that it comprises at least one pair of polarizing plates, the plates being oriented such that their polarization axes are perpendicular to each other. This composite is orientable, the bisecting lines of said axes being orthogonal to each other, according to the orthopaedic axes of the painful point to be treated.

In practice, it is rather difficult to orient "ad hoc" the active axes of the plates precisely, bearing in mind that the composite is usually applied by the patient himself.

Accordingly it is preferred that the composite possesses at least eight active axes, wherein it is provided in association with a moveable support to be applied to the skin whereby the maximum deviation from the optimun axis of one of the active axes is 360/2n . 2 wherein n is the number of polarizing plates. For n = 4, this deviation is 20° 30', which deviation can be considered almost negligible.

If, however, a smaller deviation is required, a composite can be provided having twelve active axes by means of six polarizing plates, wherein each plate is angularly offset with respect to its adjacent plate by 30°. In this case, n = 6 and the maximum deviation is only 15°.

In practice it is desirable for the composite to be fastened to an adhesive tape backing or similar system, whereby the patient has only to remove a protective layer, e.g. siliconed paper, and apply the composite adhesively to the skin in any desired orientation. Examples of such a non-adhesive support system are a wrist band and an earring.

The present invention will now be described, by way of example, with reference to the accompanying drawings, in which:-

Figures 1 and 2 show the optimum points, i.e. orthopaedic axes, of the human body over which the composite of the present invention should be applied in order to obtain maximum pain-relieving effect;

Figures 3, 4, 5 and 6 show respectively two polarizing plates with their polarization axes perpendicular to each other, an elementary composite formed from the two plates as seen in vertical elevation, and finally the same elementary composite as seen in plan with the active axes thereof coinciding with the polarization axes of the polarizing plates;

Figure 7 shows an exploded view of a composite formed from four polarizing plates, the first two plates and the last two plates constituting an elementary composite as shown in Figure 6, but with the two elementary composites being angularly offset with respect to their polarization axes by 45° in order to generate eight active axes oriented at 45° to each other;

Figures 8 and 9 respectively represent in vertical elevation the composite of Figure 7 with its active axes;

Figures 10, 11 and 12 show three elementary composites, as illustrated in Figure 5, wherein each one is fixed with respect to the other, but oriented at an angle of 30° to each other;

Figure 13 shows in elevation a composite formed from the elementary composites of Figures 10 to 12;

Figure 14 shows the active axes of the composite of Figure 13;

Figures 15 and 16 show in perspective two earrings to be fastened to a lobe of an ear AU, each earring comprising a pain-relieving composite as shown in Figures 8 and 13, respectively;

Figures 17, 18 and 19 show respectively a perspective view of a pain-relieving wrist band incorporating a composite of the invention, an exploded view of a composite as shown in Figures 3 to 6 adapted to fit such a wrist band, and, in elevation, the complete composite of Figure 18;

Figures 20, 21, 22, 23 and 24 illustrate a variation of the composite of Figures 10 to 14 in the form of an internal disc and two concentric rings; and

Figure 25 schematically illustrates the presumed mode of operation of the pain-relieving composite of the present invention.

Referring firstly to Figures 3 to 6, two plates $PS^1$ and $PS^2$ in the form of small discs are shown, the plates being made of a polarizing material, desirably a film of Polaroid (trade mark) material, with the polarization axes $LN^1$ and $LN^2$ being perpendicular to each other, whereby on superimposition of the plates to form a composite PI of Figure 5 the active axes $AS^1$, $AS^2$, $AS^3$ and $AS^4$ thereof are formed from the bisecting lines of the two polarization axes $LN^1$-$LN^2$ of the two superimposed plates.

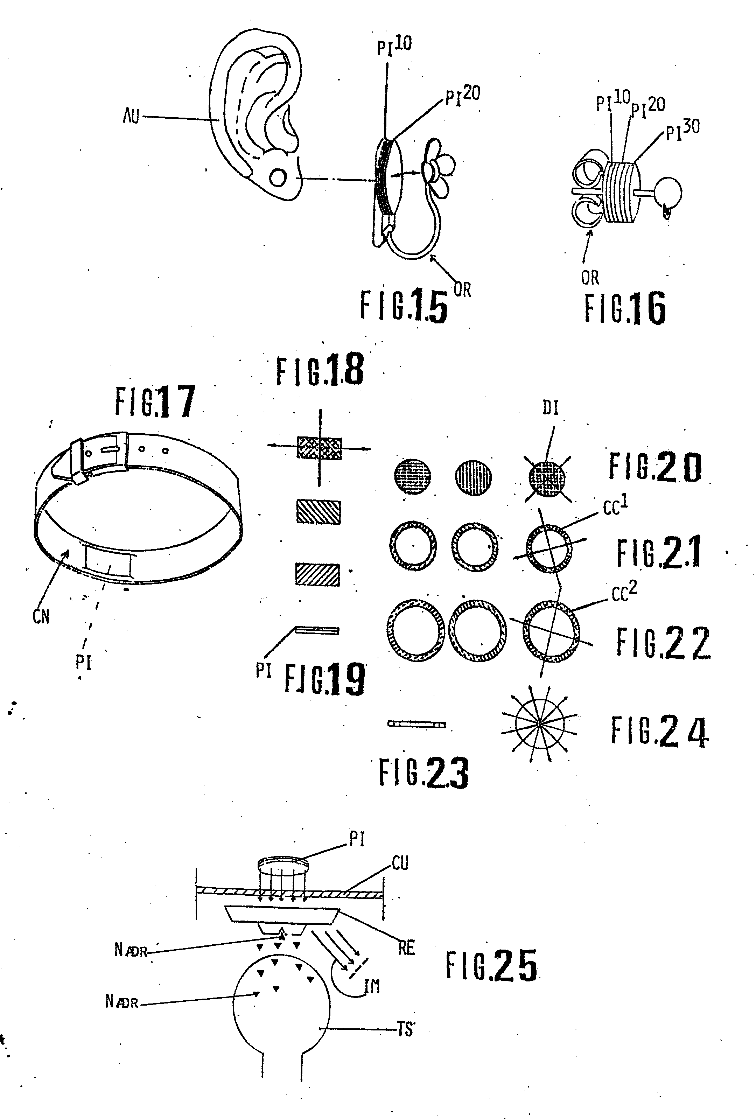

For maximum effect, the composite should be oriented so that one of its active axes ($AS^1$-$AS^4$) coincides as closely as possible with one of the axes shown in Figures 1 and 2. If a wrist band CN is used (see Figure 17) no orientation problem exists since the composite used in such a wrist band, e.g. that of Figure 5, is held by the wrist band in a fixed orientation, and thus can be pre-set in order to obtain maximum effect. When incorporated in an adhesive tape system, it is possible to print thereon the active axes of the composite so that the patient or the doctor can correctly orient the composite for maximum effect. Of course, with a wrist band the composite is pre-set according to one of its axes $AS^1$...$AS^4$.

The polarizing material may be cut into any suitable shape e.g. discs, squares or rectangles. Conveniently, the diameter of each disc is about 10 mm with a plate thickness of from 0.2-0.7 mm. The transmittance of each plate may vary from 10% to 40% or more.

Practical tests have shown that if one of the illustrated composites is applied to any painful point of the body and if the composite is so oriented that one of its active axes (as defined by one of the bisecting lines of the polarization axes of the plates) be orthopaedically oriented (as defined with reference to Figures 1 and 2) a relatively rapid relief of the pain is obtained. It has been found that the same effect may be obtained by using a composite where superimposed polarizing plates surround, rather than overlie each other, as for example shown in Figures 20 to 24 where an inner disk DI is surrounded by two concentric rings $CC^1$ and $CC^2$. Referring to Fig. 10 to 14 for best comprehension, also in this case, 12 active axes are provided, so that this concentric composite is analogous to that one of Fig. 10 to 14. Since the active life of each polarizing plate, and hence the resulting composite, is believed to be unlimited, the composites of the invention may be incorporated into an adhesive tape system so that each composite can be used not just once, but many times.

As will be clear from the foregoing, when a composite according to Figures 3 to 6 is used the maximum deviation from its optimum orientation is 360/2n . 2, wherein n is the number of plates, which in this case is two so that the maximum deviation is 360/2.2.2 = 45°.

According to a further aspect of this invention, instead of two plates as described above, four plates can be used in the form of two pairs of plates $PI^{1a}$ and $PI^{2a}$, each pair PI being as shown in Figure 5, but with the pair $PI^{2a}$ being oriented at 45° to the pair $PI^{1a}$. In this case, a composite is obtained wherein its active axes are disposed at 45° to each other, and therefore the maximum deviation for the composite of Figures 8 and 9 is 22° 30' since n in the formula 360/2n.2 is now 4.

If it is desired to decrease this deviation still further, three pairs of plates $PI^{1a}$, $PI^{2a}$ and $PI^{3a}$ can be used as shown in Figures 10 to 14, each pair being off-set by 30° so that the bisecting lines $AS^1$-$AS^4$; $AS^{1a}$-$AS^{4a}$; $AS^{1b}$-$AS^{4b}$ are similarly disposed at 30° to each other. In this case, n in the formula 360/2.n.2 is 6 giving a deviation of 15°.

In order to avoid any possible degredation of the polaroid material as a consequence of contact with human perspiration, the outer surface of the composite can be varnished so as to render it impermeable to moisture.

Although for general application an adhesive tape-based system is desirable, any mode of application of the described composites to the human body is possible. In Figures 15 and 16, for example, composites of the types shown in Figures 8 and 13 can be incorporated into an earring, of either a special or conventional kind, for affixing to the lobe AU of an ear for use in so-called ear therapy.

There now follows a brief discussion of the possible mode of pain-relieving action effected by the composites of the present invention.

For an understanding of the mode of operation of the described composites, use is made of the hypothesis that noradrenaline (NADR) is involved in the sensing of pain by perhipheral pain receptors, which receptors may be in either one or two conditions, namely:-

1. Having a normal discharge threshold in the presence of an increased quantity of NADR - (sympathetic hypertone), and

2. Having a lowered discharge threshold in the presence of physiological amounts of NADR - (sympathetic normotone).

Because of the presence of the composite of the invention a raising of the discharge threshold of the receptors is believed to take place, whereby the pain impulses from receptors in either conditions 1 or 2 above are not initiated by the endogenous NADR locally present.

The operation of the above hypothesis is illustrated in Figure 25 wherein TS shows a sympathitic terminal, NARD, the noradrenaline, RE the pain receptor, PI the composite of the invention oriented as described above, CU the skin and IM the pain impulses. As will be clear, the pain sensations are decreased by the presence of the composite PI, since the locally-present NADR causes no discharge from the receptor since its discharge threshold has been raised because of the presence of the composite PI. It is generally accepted by the scientific community that NADR is a mediator for the sensation of pain. It is therefore on the basis of this hypothesis that the raising of the discharge threshold of receptors in the presence of NADR at any point of the human body causes a decrease in the perceived sensation of pain.

The described hypothesis is supported by the observation that for a number of conditions for which patients have been successfully treated with a sympathetic regional block with guanethidine - (this being a known sympathicolytic drug used for pain relief in the treatment of e.g. Sudeck's disease, causalgia, some cervicobrachialgies and sciatic algosyndromes) similar effects have also been observed after the application of composites of the invention to the appropriate regions of the body.

In particular, it has been noticed that for patients suffering from sciatica in one of the lower limbs a significant decrease in the strength generated by the extensor muscle of the wrist on the same side has been observed, e.g. right sciatica - right wrist.

The guanethidine block used in order to achieve some relief of sciatic pain produced in these patients a clear, although temporary (3-4 days) renewal of the strength of the wrist extension.

This effect has also been recently verified in the same patients as a result of the simple application of some of the composites of the invention as described above at various points of the foot or leg.

It may thus be concluded that the described composites have a mode of action similar to that of a guanethidine block, but most probably not by removing NADR (since this requires at least 10 minutes), but by raising relatively rapidly the receptor discharge threshold in the cutaneus zones lying immediately thereunder.

The logical inference of these results is that where composites of the present invention are applied to any part of the body a relatively rapid relief of topical pain is achieved by virtue of the raising of the discharge thresholds of NADR of the receptors locally present.

The activity of the described composites is considered to be based upon terrestrial magnetism as well as upon other forms of radiation present on the earth.

As is known, the terrestrial magnetic field may be resolved into two components, one vertical that reaches a maximum at the terrestrial poles (0.7 gauss) and zero at the equator, and one horizontal component that is at a maximum at the equator - (0.33 gauss) and zero at the poles. By exploiting these two components and by raising the receptor discharge threshold, with respect to NADR, as described above, the composites of the invention are effective at any point on the earth's surface, and theoretically at least their operating life is unlimited.

In the foregoing, reference has always been made to the orthogonal orientation of the two superimposed polarizing plates of a pair, in order to obtain maximum effect. A reduced effect can however be obtained with the relative orientations of the two plates being different from 90°, and it is to be understood that such a modification is within the compass of the present invention.

It may be that the described polarizing plates in fact exploit light radiation in order to achieve their effect. In that event, provision should be made for the passage of light through the composite, for example where an adhesive tape system is used by providing an opening therein. Alternatively, a transparent material can be used for the adhesive tape system.

## Claims

1. A pain-relieving composite ( PI) with a relatively rapid action characterised in that it comprises at least one pair of plates (PS¹, PS²) of polarizing material having their polarization axes (LN¹, LN²) being perpendicular to each other and orientable so that the bisecting lines (AS¹-AS⁴) of the said axes, which lines constitute the active axes of the composite, can be arranged orthogonally with each other according to the orthopaedic axes of the point to be treated.

2. A composite according to claim 1 characterised in that it presents at least eight active axes and in that it is adapted to be mounted on a moveable support to be applied to the skin, whereby the maximum deviation between the optimum orthopaedic axis and one of the active axes is defined as $360°/2.n.2$, wherein n is the number (4) of polarizing plates in the composite.

3. A composite according to claim 2 characterised in that it is formed by six plates, each plate being angularly off-set with respect to an adjacent plate by 30° so as to provide twelve active axes, wherein the maximum deviation is 15°.

4. A composite according to any one of the preceding claims characterised in that the composite is attached to an adhesive tape, the tape having printed thereon the active axes of the composite.

5. A composite according to any one of claims 1 to 3 characterised in that the composite is attached to an adhesive tape system, the system being adapted to allow repetitive application of the composite to the point to be treated.

6. A composite according to any one of claims 1 to 3, characterised in that it forms part of an earring ( OR).

7. A composite according to claims 1 to 3, characterised in that the composite forms part of a wrist band (CN ).

8. A composite according to any one of the preceding claims characterised in that the composite consists of an elementary composite in the form of a central disc (DI) and one or more further elementary composites in the form of concentric rings (CC'-CC')so oriented to provide four, eight, twelve or more active axes, wherein the plates of the composite are coplanar.

9. A composite according to claim 5 characterised in that either the adhesive tape system is provided with an opening in order to allow free passage of light radiation therethrough, or the adhesive tape system is constructed out of transparent material having the adhesive on one surface thereof.

**0 210 321**

E196

FIG.1     FIG.2

FIG.3   LN¹, LN¹, PS¹

FIG.4   LN², LN², PS²

FIG.5   PI

FIG.6   AS¹, AS², AS³, AS⁴, PI

FIG.10   AS¹, AS², AS³, AS⁴, PI¹⁰

FIG.11   AS¹⁰, AS²⁰, AS³⁰, AS⁴⁰, PI²⁰

FIG.12   AS¹¹, AS²², AS³³, AS⁴⁴, PI³⁰

FIG.7   PI¹⁰, PI²⁰

FIG.9   AS¹, AS², AS³, AS⁴, AS⁵, AS⁶, AS⁷, AS⁸

FIG.8   PI¹⁰, PI²⁰

FIG.13   PI¹⁰, PI²⁰, PI³⁰

FIG.14   30°

FIG.15

FIG.16

FIG.18

FIG.17

FIG.19

FIG.20

FIG.21

FIG.22

FIG.23

FIG.24

FIG.25

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 453 654 (NOGIER)<br>* Page 5 * | 1-9 | A 61 N  5/00 |
| X,D | EP-A-0 065 473 (BRICOT)<br>* Claims 1-6 * | 1-9 | |

---

**TECHNICAL FIELDS SEARCHED (Int. Cl.4)**

A 61 N

The present search report has been drawn up for all claims

| Place of search | Date of completion of the search | Examiner |
|---|---|---|
| THE HAGUE | 24-03-1986 | ONILLON C.G.A. |